# EUROPÄISCHE PATENTANMELDUNG

(11) **EP 1 975 245 A1**
(43) Veröffentlichungstag der Anmeldung: **01.10.2008**
(21) Anmeldenummer: 07006352.4
(22) Anmeldetag: 28.03.2007
(51) Int. Cl.: C12Q 1/68

(54) **Gen-Profiling zur Auswahl von politischen Kandidaten, kommerzielle Nutzung von Politikern**

(71) Anmelder: Greenpeace e.V., 22767 Hamburg (DE)
(72) Erfinder: Then, Christoph, Dr., 22767 Hamburg (DE)

(57) **Zusammenfassung**

Die Erfindung betrifft ein Verfahren zur Überprüfung von Menschen auf ihre Eignung als Politiker umfassend die folgenden Schritte:
a) Erstellen von Gen-Profilen (GP) mit Hilfe von geeigneten Referenzpopulationen (RP)
b) Ermittlung besonders geeigneter Gen-Marker (GM), ausgewählt durch Abgleich der Gen-Profile (GP) der Referenzpopulationen (RP)
c) Sequenzierung von Genen, die den jeweiligen Gen-Markern (GM) zugeordnet werden können, um deren Spezifität zu bestimmen
d) Abgleich der Genprofile (GP) mit dem Genprofil von politischen Kandidaten (PK) oder von Personen, die möglicherweise als politische Kandidaten geeignet sind
e) Abgleich der Ergebnisse aus den Verfahren von a-d mit weiteren Kriterien, um das Ergebnis des Gen-Profiling abzusichern (wie Meinungsumfragen).

## Beschreibung

### Hintergrund der Erfindung:

Diese Erfindung nutzt die Entwicklung im internationalen Patentrecht, zunehmend die belebte Natur, Entdeckungen und biologische Verfahren dem Patentrecht zugänglich zu machen. So wurden beispielsweise 2007 ein Verfahren zur Züchtung normaler (und genmanipulierter) Kühe erteilt (EP 1330552)', 2006 ein Verfahren zur Züchtung von gentechnisch nicht verändertem Salat (EP 1179089).

Bei diesen >Erfindungen< wurden die natürlicherweise vorkommenden Gene in Pflanzen und Tieren genutzt, diese Gene wurden dabei nur untersucht und als Selektionsmerkmal genutzt, sie werden aber nicht gentechnisch verändert. Das Europäische Patentamt prüft nun im Fall eines Patentantrages auf Brokkoli (EP1069819) sogar ganz generell Patente auf im wesentlichen biologische Verfahren zur Züchtung von Pflanzen und Tieren durch die Große Beschwerdekammer erlauben zu lassen.

Diese Entwicklung eröffnet auch die Möglichkeit, die wirtschaftliche Verwertung von entsprechenden Genen und Genprofilen von Menschen zum Patent anzumelden, um diese bei der Auswahl bestimmter gewünschter human ressources nutzen zu können. Diese Patentanmeldung setzt hier an und beschreibt auf dem technischen und rechtlichen Niveau bereits erteilter Patente ein technisches Verfahren zur Auswahl von Politikern (>marker assisted breeding<).

In einer Welt von zunehmender Komplexität erscheint es besonders problematisch, dass PolitikerInnen immer noch nicht aufgrund von wissenschaftlich überprüfbaren Fakten sondern aufgrund von meist weder qualitativ noch quantitativ überprüfbaren Eigenschaften aufgestellt, gewählt und in Regierungsämter berufen werden. Hier setzt diese Erfindung an: Es ist allgemein bekannt, dass Gene auch bei. Politikern Verhalten und Eigenheiten mitbestimmen können. Bei der vorliegenden Erfindung kaommt es darauf an, diesen genetischen Einfluss so zu erfassen, dass dieser kommerziell nutzbar gemacht werden kann.

Der allgemeine wissenschaftliche Hintergrund der Erfindung kann u.a. in Richard Dawkins ,,Das egoistische Gen" (Dawkins, R, 1976, "The Selfish Gene", New York/Oxford: Oxford University Press) nachgelesen werden. Hier wird ausführlich dargelegt, dass menschliche Individuen nichts anderes wären als Roboter, die von ihren Genen gesteuert werden und dass auch soziale Interaktionen vollständig auf genetische Veranlagungen zurückgeführt werden können. Demnach sind Menschen nichts anderes als die "Sklaven ihrer Gene". Bisher wurde diese wissenschaftliche Analyse aber nicht dazu herangezogen, konkrete und wirtschaftlich verwertbare Aussagen gerade über die Personen zu machen, die als PolitikerInnen für die Zukunft der Gesellschaft von zentraler Bedeutung sind.

Methoden zur Erfassung (Gen-Profiling, Marker Assisted Breeding), Analyse und kommerziellen Verwertung des Erbgutes von Politikern werden hier erstmals beschrieben. Mit der beschriebenen Methode kann das Erbgut jedweden Politikers erfasst werden, soweit es kommerziell nutzbar ist. Die mögliche Verwendung reicht über die Aufstellung von Kandidaten vor Wahlen, die gezielte Auswahl für Regierungsämter, das Vorab-Screening von Kandidaten für das Amt von Bundeskanzler und Bundespräsident, EU-Kommissaren und anderen Staatsämtern im In- und Ausland.

Bei der Erstellung der Patentanmeldung wurde der ethische und rechtliche Rahmen der europäischen Patentgesetze strikt beachtet. Konkrete wirtschaftliche Anwendungen werden in den Ansprüchen und der Beschreibung der Patentschrift benannt.

Die Methode beruht auf einem Abgleich von Gen-Profilen eines repräsentativen Ausschnitts der Bevölkerung mit den Profilen von PolitikerInnen oder solchen, die gerne Politiker werden wollen.

Besondere Relevanz hat das Verfahren unter anderem (nicht abschließende Aufzählung):
· zur Auswahl von Politikern, die für bestimmten Regionen besonders regionaltypische Merkmale erfüllen müssen (>Regionaltyp<)
· zur Auswahl von Politikern, die für bundesweite oder europaweite Aufgaben möglichst wenig,regionaltypische Merkmale erfüllen sollen (>Globaltyp<)
· zur Auswahl von Politikern mit besonders ausgeprägten und ungewöhnlichen Charakterzügen (<Charaktertyp<) Zur Auswahl von Politikern mit möglichst wenig gering ausgeprägtem Charaktertyp (>Flexibler Typ<)
· Zur Auswahl von Politikern, die für bestimmte Wählerschichten oder Themenspektren besonders geeignet sind (>inhaltlich gebundener Typ<)
· Zur Auswahl von Politikern, die zu allen Themen und Wählerschichten gleichermaßen eingesetzt werden können (>one-size-fits all Typ<)

Genutzt werden kann das Verfahren aber auch zur Auslese von Politikern, die darüber hinausgehende Eigenschaften oder andere besondere Eignungen aufweisen sollen.

Bisher war die Auswahl von Politikern im wesentlich durch Zufall, interne Seilschaften, Parteizugehörigkeit; Traditionen und Meinungsumfragen'gestützt. Hier wird erstmals ein wissenschaftlich basiertes Verfahren zur Auswahl der Politiker beschrieben, das mit konventionellen Methoden wie Meinungsumfragen kombiniert werden kann und zu einer erheblichen Verbesserung der Qualität und der Effektivität der Auswahl von PolitkerInnen führen kann.

Der wirtschaftliche Nutzen des Verfahrens und dessen kommerzielles Potential kann insbesondere verdeutlicht werden, wenn man sich vor Augen führt, welcher wirtschaftlicher Schaden durch ungeeignete Politiker im Laufe der letzten hundert Jahre schon angerichtet wurde. Auch in Bezug auf die derzeitige Bundesregierung oder die derzeitig amtierende Europäische Kommission könnte das Verfahren genutzt werden, um die Qualität der human ressources in Bezug auf die aktuellen politischen Aufgaben zu optimieren.

Die kommerzielle Nutzung von Politikern erhält mit dieser Erfindung eine völlig neue Dimension: Die Eigenschaften von Politikern werden qualitativ und technisch klar definierbar und damit zur echten Erfindung und zum patentgeschützten Produkt. Eine breitgefächerte Palette neuer und innovativer Anwendungen ist das Ergebnis dieser technischen Entwicklung. Es ist zu erwarten, dass nicht nur die Demokratie insgesamt, sondern insbesondere bestimmte Wirtschaftszweige von dieser Erfindung profitieren werden.

Damit einhergehend ist es auch erstmal möglich, politische Erfolge, gewonnene Wahlen oder steigende Umfragewerte durch entsprechende Lizenzverträge kommerziell nutzbar zu machen. Damit können politische Erfolge sogar zum Gegenstand börsennotierter Wertschöpfung gemacht werden. So wird es für einen größeren Kreis interessierter Anleger erstmals möglich, gezielt in bestimmte Karieren oder politische Programme zu investieren.

### Beschreibung:

Aus dem Stand der wissenschaft ergibt sich, dass Politiker sowohl durch ihre einzelnen Gene als auch durch das Zusammenspiel ihrer Gene in bestimmten Rahmen beeinflusst und charakterisiert werden können. Ein wesentlicher Bestandteil dieser' Erfindung ist es, sich nicht auf einzelne Erbanlagen zu beschränken, sondern das gesamte Genom zur Basis der kommerziellen Nutzung zu machen.

Zur Durchführung der Erfindung wird biologisches Material an geeigneter Stelle mit geeigneter Qualität (wie Speichelproben, Haare mit Haarwurzeln, Gewebeproben aller Art) gesammelt und nach bekannten technischen Verfahren behandelt, so dass das Muster der einzelnen Gen-Abschnitte ein für.die jeweilige Person typisches Gen-Profil (Fingerabdruck) ergibt. Nachfolgend werden entsprechende. Gensequenzen analysiert und durch Abgleich mit weiteren Genprofilen überprüfbare Aussagen über die Eignung von PolitikerInnen abgeleitet (sog. marker assisted breeding).

Neu ist die Verknüpfung der verschiedenen Analyseverfahren zu einem System der Identifizierung von erblichen Merkmalen bei Politikern und ihrer Auswahl wie etwa im Vorfeld von Wahlen. Das hier offenbarte Verfahren kann jedoch auch zur Auswahl wirtschaftlich besonders interessanter Pflanzen und Tiere, Mikroorganismen und anderer Lebewesen genutzt werden, die einer wirtschaftlichen Verwertung zugänglich gemacht werden sollen.

Offenbart wird insbesondere die Nutzung des so genannten genetischen Fingerabdruckes (gene profiling) in Verbindung mit Genanalyse im einem definierten Umfeld zur Ermittlung von relevanten Eigenschaften von Politikern. Als Dienstleistung kann das Verfahren unter anderem'großen Parteien angeboten werden, deren Finanzierung und politischer Erfolg bekanntermaßen insbesondere von der Auswahl geeigneter Persönlichkeiten (human ressources) abhängt, diese sind das >Kapital< der politischen Parteien.

Offenbart werden hier auch beispielhaft geeignete genetische Marker und Gensequenzen zur Auswahl von PolitikerInnen des >Globaltyps<.

Offenbart werden auch Verfahren zur Auswahl geeigneter definierter Umweltbedingungen, in deren Rahmen die offenbarten Verfahren effektiv eingesetzt werden können.

Die hier patentierten technischen Verfahren umfassen folgende Schritte:
1. Auswahl geeigneter Merkmale, mit denen entsprechende human ressources in Bezug auf die jeweiligen kurz-, mittel- und langfristigen politischen Ziele ausgewählt werden sollen.
2. Ermittlung der jeweiligen Referenzpopulation
3. Auswahl geeigneter Markerregionen durch Abgleich von Genprofilen
4. Sequenzierung besonders auffälliger Genregionen zur Ableitung spezifischer Aussagen
5. Abgleich der Genprofile von jeweils zur Verfügung stehenden politischen Kandidaten, bzw. Ermittlung von Personen, die als weitere Kandidaten in Frage kommen
6. Heranziehen weiterer Auswahlverfahren wie Umfragen, Medientauglichkeit, politische Bildung, um das Ergebnis des Gen-Profiling zu überprüfen.
7. Standardtisieren der Methode durch mehrere Wiederholungen um ein jederzeit verfügbares und effektives Testsystem für multiple Gelegenheiten zu etablieren (>Fast-Screen<)

In einer weiteren Variante kann das Verfahren herangezogen werden, um weiteres wirtschaftlich interessantes biologisches Material (wie Tiere und Pflanzen) zu selektieren.

### Beispiele:

### Beispiel 1: Nutzung einer Gendatenank zur Ermittlung von >Globaltypen< (bzw >one size fits all< typ):

Da seit mehreren Jahren bereits genetische Fingerabdrücke zu ganz unterschiedlichen Zwecken gewonnen werden (Zur Überprüfung von verwandtschaftsverhältnissen, Ermittlung von Kriminellen) existieren erhebliche Datenmengen. Dies können zur Ermittlung des >Globaltyps< wie folgt genutzt werden:
1. Abgleich einer großen Menge von genetischen Profilen, um besonders häufig auftretende Marker-Regionen zu ermitteln
2. Sequenzierung der zugehörigen Gene und Abgleich der Gensequenzen mit weiteren Datenbanken, um mögliche biologische Funktionen zu ermitteln.
3. Vergleich mit Genprofilen von Referenz-Populationen aus definierten Umwelten (z.B. Passauer Nibelungenhalle, Münchner Oktoberfest, Hamburger Reeperbahn, Berliner Taxifahrer, EU-Konferenzen in Brüssel) um das Gen-Profiling weiter zu gewichten und qualifizieren
4. Abgleich mit Markern von geeigneten Kandidaten bzw. Auswahl von Personen, die als Kandidaten geeignet sein könnten.

Im vorliegenden Fall wurde folgende Markerregion beispielhaft untersucht:
Laut John M. Butler, Forensic DNA Typing, kommt das Allel 8 des Mikrosatelliten TPOX bei ca 50% der kaukasischen, 37% der afroamerkanischen und 47% der hispanischen Bevölkerung vor. Damit erscheint dieses Allel besonders geeignet, im Rahmen von Verfahren zur Überprüfung von Menschen auf ihre Eignung als Politiker verwendet werden zu können (zum Beispiel im Rahmen sogenannter Differenzverfahren).
Deswegen wurde in einem nachfolgenden Schritt wurden folgende zugehörige Gensequenzen dieser Markerregionen ermittelt. Dazu wurde eine zufällige Referenzpopulation von 16 Personen des kaukasischen Typs ausgewählt, bei denen das Vorhandensein des Allels vorab im einem Genprofil bestimmt wurde. Dabei zeigt sich, dass bei allen 16 Personen die analysierte Gensequenz zu 100% übereinstimmte.

Nachfolgend ist es für den interessierten Fachmann ein leichtes, durch Abgleich entsprechender Gensequenzen mit weiteren Datenbanken auch Aussagen über deren mögliche biologischen Funktionen zu ermitteln.

Weiterhin ist es für den interessierten Fachmann jederzeit möglich, entsprechende Genprofile von Kandidaten zu erstellen, um diese mit den vorliegenden Gensequenzen abgleichen zu können.

### Beispiel 2: Ermittlung von >Regionaltyp< bzw >Charaktertyp< durch gezielten Vergleich mit geeigneten Referenzpopulationen

Im vorliegenden Fall soll das hier offenbarte Verfahren dazu herangezogen werden, um beispielhaft die Eignung von Politikern für das Amt des Bayerischen Ministerpräsidenten zu ermitteln. Insbesondere Bayerische Politiker zeichnen sich durch eine hohes Maß an sogenannter Bodenständigkeit aus, das hier naturwissenschaftlich genauer als bisher beschrieben und dadurch auch nutzbar gemacht werden kann.

Als geeignete Referenzpopulation sind die Teilnehmer des alljährlich in der Passauer Nibelungenhalle stattfindenden politischen Aschermittwochs geeignet.¹
¹ Ungeeignet erscheint dagegen das Münchner Oktoberfest, da nicht alle Personen, die hier phänotypisch als Bayern auftreten (Lederhose, Dirndl) auch wirklich dem bayerischen Regionaltyp zuzuordnen sind. Vielmehr handelt es sich dabei oft um Italiener, Japaner, Amerikaner, Australier, Nord- und Ostdeutsche. Dagegen wäre das Oktoberfest als Referenzpopulation sehr wohl geeignet, um den >Globaltyp< zu definieren.

Als, mögliche, Ausgangssubstanzen für ein gezieltes >Biöprospekting< in der Nibelungenhalle könnten folgende Proben dienen: Taschentücher oder Servietten mit verschiedenen Körpersekreten, Urinroben (z.B. gewonnen durch heimlich in die Urinale eingebaute Probensammler), Blutproben (z.B. getarnt a1s Promille-Test), Haare, Schuppen, Bestecke mit Speichelresten, Spuckprobenasservate, Gewebereste.

Die Proben würden im Labor nach folgende Stufen zielführend verwendet:
1. Erstellung von Genprofilen
2. Abgleich der Genprofile nach besonders häufigen Markern
3. Abgleich der identifizieren Marker mit Profilen des Globaltyps um möglichst regional spezifische Genmarker zu identifizeren (sog. Differenzverfahren)
4. Sequenzierung der identifizierten Gene, um eventuelle biologische Relevanz zu erfassen
5. Abgleich der Gene mit geeigneten Kandidaten
6. Heranziehen weiterer Qualitätsstandards um das Ergebnis des Genprofiling abzusichern

### Beispiel 3: Ermittlung eines besonderen >Charaktertyps< durch gezielten Vergleich mit geeigneten Referenzpopulationen

Im vorliegenden Fall soll das hier offenbarte Verfahren dazu herangezogen werden, um beispielhaft Politiker zu finden, die sich durch besonderen Wagemut auszeichnen.

Als geeignete Referenzpopulation könnten hier die Personen ausgewählt werden, die auf großen Achterbahnen oder bei ähnlichen Einrichtungen, deren Nutzung besondere Risikobereitschaft voraussetzt, angetroffen werden.

Als mögliche Ausgangssubstanzen für ein gezieltes >Bioprospekting< könnten folgende Proben dienen: Kaugummi, Biergläser, Taschentücher mit verschiedenen Körpersekreten, Blutproben (z.B. getarnt als Promille-Test), Haare, Schuppen, Spuckprobenasservate, Gewebereste.

Die Proben würden im Labor nach folgende Stufen zielführend verwendet:
1. Erstellung von Genprofilen
2. Abgleich der Genprofile nach besonders häufigen Markern
3. Abgleich der identifizieren Marker zum Beispiel mit Profilen des >one size fits all Typs<, um möglichst spezifische Genmarker für >Wagemut< zu identifizeren (sog. Differenzverfahren)
4. Sequenzierung der identifizierten Gene, um eventuelle biologische Relevanz zu erfassen
5. Abgleich der Gene mit geeigneten Kandidaten
6. Heranziehen weiterer Qualitätsstandards um das Ergebnis des Genprofiling abzusichern
² wie Dialekt, Trinkfestigkeit, Jodelpotential

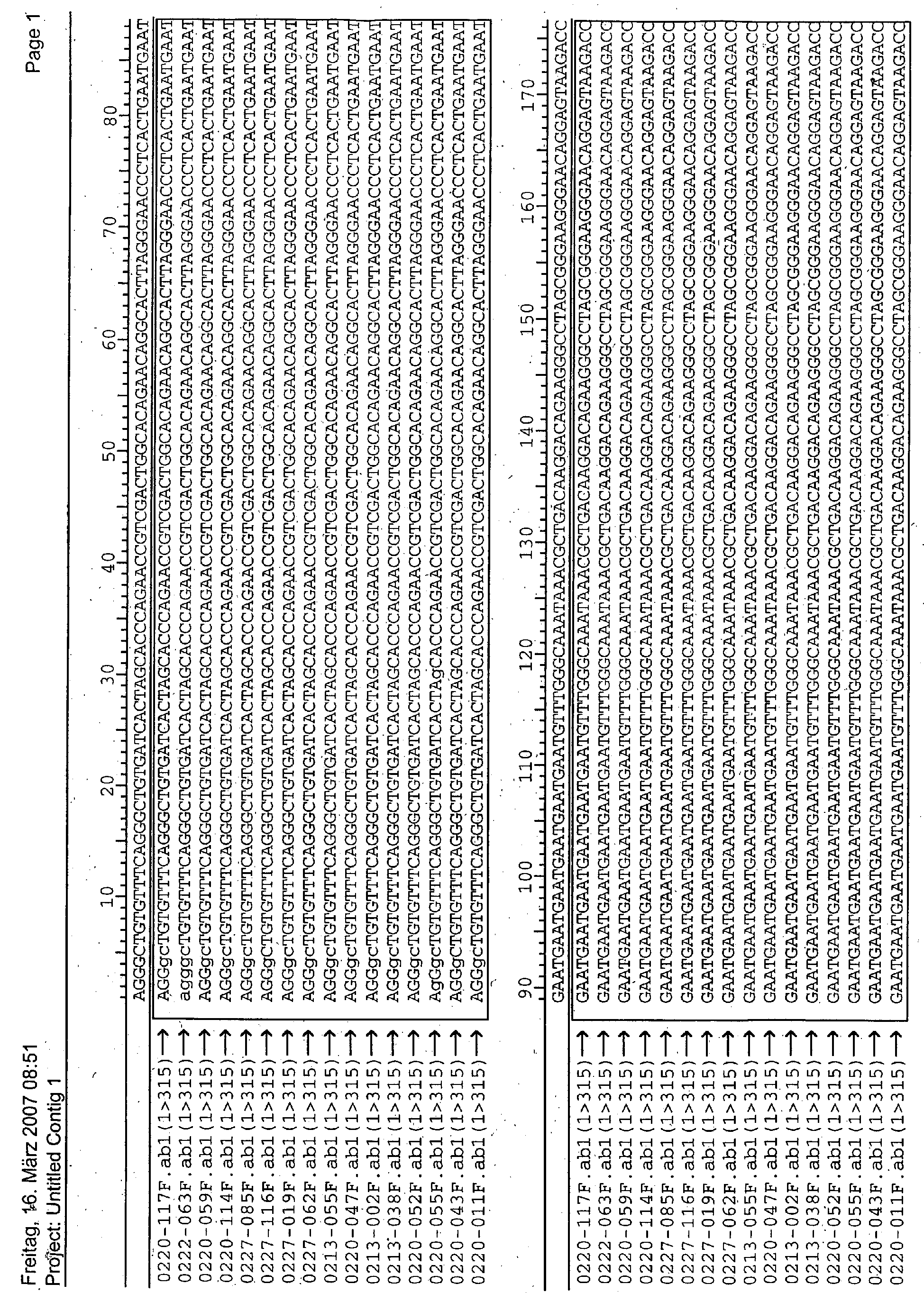

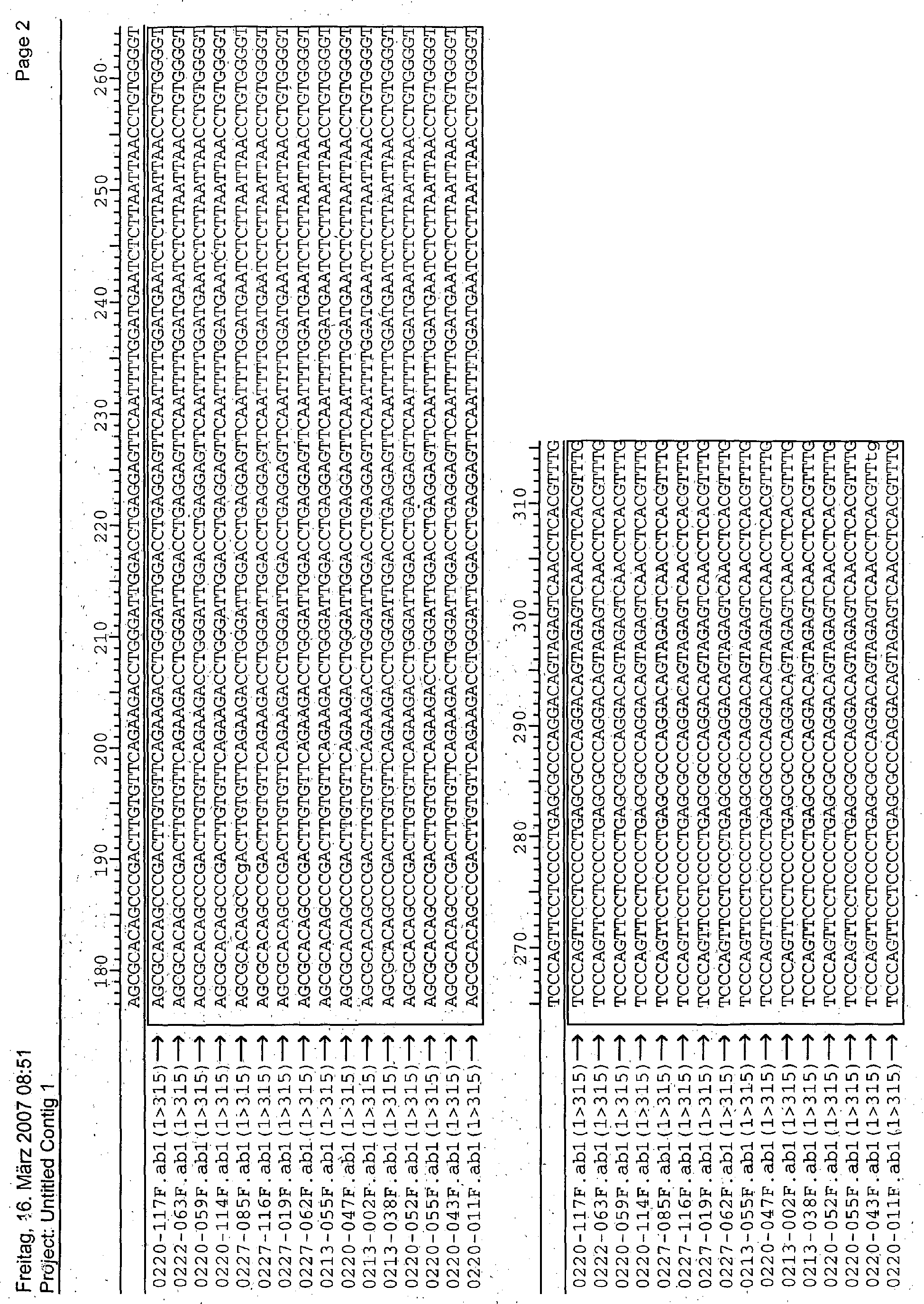

## Patentansprüche

1. Verfahren zur Überprüfung von Menschen auf ihre Eignung als Politiker umfassend die folgenden Schritte:
a) Erstellen von Gen-Profilen (GP) mit Hilfe von geeigneten Referenzpopulationen (RP)
b) Ermittlung besonders geeigneter Gen-Marker (GM), ausgewählt durch Abgleich der Gen-Profile (GP) der Referenzpopulationen (RP)
c) Sequenzierung von Genen, die den jeweiligen Gen-Markern (GM) zugeordnet werden können, um deren Spezifität zu bestimmen
d) Abgleich der Genprofile (GP) mit dem Genprofil von politischen Kandidaten (PK) oder von Personen, die möglicherweise als politische Kandidaten geeignet sind
e) Abgleich der Ergebnisse aus den Verfahren von a-d mit weiteren Kriterien, um das Ergebnis des Gen-Profiling abzusichern.(wie Meinungsumfragen).

2. Verfahren nach Anspruch 1, wobei die Referenzpopulation (RP) nach regionalen Besonderheiten und/oder nach vorbestimmten Vorlieben und/oder nach bestimmten Wählerschichten ausgewählt ist.

3. Verfahren nach einem der Ansprüche 1 oder 2, weiter umfassend den Schritt:
Auswahl von Menschen als geeignete Politiker des Globaltyps, Regionaltyps,
Charaktertyps, inhaltlichen Typs oder eines anderen vorbestimmten Persönlichkeitsprofils.

4. Politikerin ausgewählt nach einem Verfahren gemäß Anspruch 3.

5. Gen-Marker (GM) zur Bestimmung und Auswahl von Politikern, ausgewählt durch
a) Erstellen eines Gen-Profils (GP) mit Hilfe einer Referenzpopulation (RP)
b) Abgleich der Gen-Profile (GP) der Referenzpopulation (RP) und Bestimmung
besonders häufiger oder besonders auffälliger Regionen in den Gen-Profilen (GP)
c) Vergleich mit Gen-Profilen (GP) anderer Referenzpopulationen.

6. Gen-Marker (GM) nach Anspruch 5, wobei der Vergleich mit Gen-Profilen (GP) anderer Referenzpopulationen (RP) im Differenzverfahren erfolgt.

7. Menschliches Allel 8 des Mikrosatteliten TPOX zur Verwendung als Gen-Marker in einem Verfahren nach Anspruch 1-3.

8. Gensequenzen, RNA und Proteine, die mit Gen-Marken (GM) nach einem der Ansprüche 5 oder 6 korreliert werden können.

9. Menschliche Gensequenz des Allel 8 des Mikrosatteliten TPOX wie sie in Abb. 1 offenbart ist.

10. Testkit (TK), insbesondere auf Basis von ELISA Tests, zur Identifizierung von Proteinen nach Anspruch 8, zur Auswahl von Politikern.

11. Verfahren zur Erfassung, Sichtung und Bewertung des Erbgutes von
Politikern und ihre Nutzung zu politischen, wirtschaftlichen und kulturellen Zwecken.

12. Erbgut von Politikern, soweit es wirtschaftlich, nutzbar ist.

13. Verfahren zum Aufbau und Nutzung einer Datenbank mit den genetischen Daten von Politikern.

14. Verfahren nach einem der Ansprüche 1 bis 3, weiter umfassend den Schritt:
Aufstellung von auf ihre Eignung als Politiker überprüfte Menschen als Kandidaten auf Parteiveranstaltungen, Berufung von Ministern, Wahl von Ministerpräsident, Bundeskanzler, Bundespräsident oder anderer Politiker in In- und Ausland.

15. Verfahren zur Vorhersage von Wahlergebnissen, **dadurch gekennzeichnet,**
**dass** der genetische Fingerabdruck der wählenden Bevölkerung genutzt wird.

16. Eine Herde (Gruppe) von PolitikerInnen mit einer erhöhten Frequenz
eines bestimmten Politikertyps, die durch die folgende Methode
zusammengeführt wurde:
a) screening der Kandidaten nach einem Verfahren nach Ansprüchen 1 bis 3
b) Auswahl der PolitikerInnen mit ähnlichen Typenprofilen nach Anspruch 3
c) Zusammenstellung der ausgewählten PolitikerInnen zu einer "Partei".

17. Pflanzen oder Tiere oder andere Lebensformen, die nach folgenden Verfahren ausgewählt wurden:
a) Erstellen von Gen-Profilen (GP) mit Hilfe von geeigneten Referenzpopulationen (RP)
b) Ermittlung besonders geeigneter Gen-Marker (GM), ausgewählt durch Abgleich der Gen-Profile (GP) der Referenzpopulationen (RP)
c) Sequenzierung von Genen, die den jeweiligen Gen-Markern (GM) zugeordnet werden können, um deren Spezifität zu bestimmen
d) Abgleich der ermittelten Gen-Marker (GM) mit dem Genprofil von weiteren Pflanzen oder Tieren, mit dem Zweck geeignetes biologisches Material zu Zuchtzwecken zu identifizieren
e) Abgleich mit weiteren Kriterien um das Ergebnis des Gen-Profiling abzusichern (wie weitere Leistungsmerkmale).


SEQUENCE LISTING

<110>  Greenpeace e.V.

<120>  Allel 8 im System TPOX

<130>  07006352.4 - 2403

<140>  07006352.4 - 2403
<141>  2007-05-09

<160>  1

<170>  PatentIn version 3.3

<210>  1
<211>  315
<212>  DNA
<213>  Homo sapiens

<400>  1
agggctgtgt ttcagggctg tgatcactag cacccagaac cgtcgactgg cacagaacag      60

gcacttaggg aaccctcact gaatgaatga atgaatgaat gaatgaatga atgtttgggc     120

aaataaacgc tgacaaggac agaagggcct agcgggaagg gaacaggagt aagaccagcg     180

cacagcccga cttgtgttca gaagacctgg gattggacct gaggagttca attttggatg     240

aatctcttaa ttaacctgtg gggttcccag ttcctcccct gagcgcccag gacagtagag     300

tcaacctcac gtttg                                                      315